# EUROPEAN PATENT APPLICATION

(11) **EP 3 326 522 A1**
(43) Date of publication of application: **30.05.2018**
(21) Application number: 17204009.9
(22) Date of filing: 28.11.2017
(51) Int. Cl.: A61B 5/00, A61B 5/11

(54) **WEARABLE SYSTEM FOR PRESSURE ULCER PREVENTION**

(30) Priority: 29.11.2016 US 201662427542 P
(71) Applicant: Hill-Rom Services, Inc., Batesville, IN 47006-9167 (US)
(72) Inventor: KAIKENGER, Philippe, Batesville, IN Indiana 47006-9167 (US); WILLIAMSON, Rachel L, Batesville, IN Indiana 47006-9167 (US); VENTROLA, Todd Steven, Batesville, IN Indiana 47006-9167 (US); TALLENT, Dan R, Batesville, IN Indiana 47006-9167 (US); COLEMAN, II, Leigh Scott, Batesville, IN Indiana 47006-9167 (US); STEBBINS, Kristen L, Batesville, IN Indiana 47006-9167 (US); KING, Catherine M, Batesville, IN Indiana 47006-9167 (US); SALIBRA, Alisa R, Batesville, IN Indiana 47006-9167 (US); LAI, Chee Keen, Batesville, IN Indiana 47006-9167 (US)
(74) Representative: Findlay, Alice Rosemary

(57) **Abstract**

A system for preventing pressure ulcers on a patient supported on a patient support apparatus is disclosed. The system is configured to sense movement of the patient over time to determine if the movement of the patient is adequate to prevent the patient from developing pressure ulcers.

## Description

The present disclosure is related to a patient apparatus system that includes body-mounted sensors that are operable to gather data from a patient supported on a patient support apparatus. More specifically, the present disclosure relates to a system for a patient support apparatus that is operable to prevent the patient from developing pressure ulcers.

In a hospital setting, a patient may be supported on a patient support apparatus such as a hospital bed. Typically, the patient supports themselves on the hospital bed by resting on pressure areas such as, for example, their head, shoulders, elbows, hips, heels, etc. The tissue surrounding the pressure areas may experience poor blood perfusion if the patient supports themselves with the same pressure areas over an extended period of time. Each pressure area may be relieved periodically if the patient moves or is repositioned to change the pressure areas supporting the patient. However, if the patient does not move for an extended period of time, the poor blood perfusion in a pressure area may increase the probability that pressure ulcers will occur in that area.

To prevent pressure ulcers, some hospital beds capture the location of the patient's gravity center and the displacement of the gravity center. The captured information is used to indicate whether the patient has moved relative to the patient support apparatus. However, the information does not indicate whether the patient remains being supported by the same pressure areas.

The present application discloses one or more of the following features alone or in any combination.

According to a first aspect of the present disclosure, a system for preventing pressure ulcers on a patient supported on a patient support apparatus comprises a first sensor, a second sensor and means for comparing movement of the first sensor relative to the second sensor over time to determine if the relative movement of the first and second sensors is indicative of a change in an orientation of the patient or an orientation of the patient support apparatus. The first sensor may be configured to be coupled to the patient at a first location. The second sensor may be configured to be coupled to the patient at a second location.

In some embodiments, the means for comparing movement of the first sensor relative to the second sensor may include a controller including a processor and a memory. The memory may have, stored therein, a plurality of instructions that when executed by the processor cause the controller to determine a first position of the first sensor and a first position of the second sensor, determine a second position of the first sensor and a second position of the second sensor, compare the change in position of the first sensor relative to the change in position of the second sensor to determine if the patient has moved relative to the patient support apparatus during a predetermined time interval, and generate an alarm signal if the relative movement of the patient is indicative of an increased risk of the patient developing pressure ulcers due to inadequate movement of the patient during the predetermined time interval.

In some embodiments, the first sensor may include an accelerometer coupled to a hip of the patient. In some embodiments, the second sensor may include an accelerometer coupled to a shoulder of the patient.

In some embodiments, the first sensor may be configured to be coupled to the patient in a location that is spaced apart from a high-pressure area of the patient's hip. The second sensor may be configured to be coupled to the patient in a location that is spaced apart from a high-pressure area of the patient's shoulder. The plurality of instructions may further cause the controller to use data from the first and second sensors as a proxy for actual pressures acting on the patient at the high-pressure areas of the patient.

In some embodiments, the patient support apparatus may include a turn system. The plurality of instructions may further cause the controller to transmit the alarm signal to the patient support apparatus to activate a turn system included in the patient support apparatus to cause the turn system to reposition the patient relative to the patient support apparatus.

In some embodiments, the controller may be connected to a hospital network. The plurality of instructions may further cause the controller to transmit the alarm signal over the hospital network to alert a caregiver.

According to another aspect of the present disclosure, a controller of a patient support apparatus may include a processor, and a memory. The memory may have stored therein a plurality of instructions that when executed by the processor cause the controller to receive a first data set indicative of a first position of a patient supported on the patient support apparatus relative to the patient support apparatus, receive a second data set indicative of a second position of the patient relative to the patient support apparatus after a time interval, compare the second position of the patient to the first position of the patient, and generate an alarm signal if a difference between the second position of the patient and the first position of the patient is indicative of an increased risk of the patient developing pressure ulcers due to inadequate movement of the patient's limbs over the time interval.

In some embodiments, each of the first and second data sets may include information indicative of a position of the patient's hip and shoulder relative to the patient support apparatus. In some embodiments, the controller may be configured to receive data from at least one sensor that is configured to be coupled to the patient in a location that is spaced apart from a high-pressure area of the patient. The plurality of instructions may further cause the controller to use the data from the at least one sensor as a proxy for a pressure acting on the patient at the high-pressure area.

In some embodiments, the high-pressure area of the patient may include one or more of the patient's hip and the patient's shoulder. In some embodiments, the time interval may be about two hours.

In some embodiments, the patient support apparatus may further includes a turn system. The plurality of instructions may further cause the controller to transmit the alarm signal to the patient support apparatus to activate a turn system included in the patient support apparatus to cause the turn system to reposition the patient relative to the patient support apparatus.

In some embodiments, the controller may be connected to a hospital network. The plurality of instructions may further cause the controller to transmit the alarm signal over the hospital network to alert a caregiver. In some embodiments, comparing the second position of the patient to the first position of the patient may include comparing movement of a first sensor relative to a second sensor over time to determine if the relative movement of the first and second sensors is indicative of a change in an orientation of the patient.

A method of preventing a patient from developing pressure ulcers is disclosed which includes a number of steps. The method may include receiving a first data set indicative of a first position of a patient supported on a patient support apparatus relative to the patient support apparatus, receiving a second data set indicative of a second position of the patient relative to the patient support apparatus after a time interval, comparing the second position of the patient to the first position of the patient, and generating an alarm signal if the comparison between the second position and the first position during the time interval is inadequate for preventing the patient from developing pressure ulcers.

In some embodiments, the method may further include coupling an accelerometer to the patient in a location that is spaced apart from a high-pressure area of the patient. In some embodiments, the method may further include coupling a first accelerometer to a hip of the patient and coupling a second accelerometer to a shoulder of the patient.

In some embodiments, the method may further include activating a turn system configured to reposition the patient relative to the patient support apparatus in response to the alarm signal being generated. In some embodiments, the method may further include transmitting the alarm signal to a remote computing device via a hospital network.

The invention will now be further described by way of example with reference to the accompanying drawings, in which:
Fig. 1 is a perspective and diagrammatic view of a patient support apparatus system including a patient support apparatus, a number of body-mounted sensors situated on a patient supported on the patient support apparatus and, schematically shown, a controller in communication with the body-mounted sensors and the patient support apparatus to monitor the patient's position relative to the patient support apparatus;
Fig. 2 is a diagrammatic representation of the patient support apparatus system of Fig. 1, the illustrative patient support apparatus is connected to a hospital network of a hospital and in wireless communication with the body-mounted sensors situated on the patient supported by the patient support apparatus;
Fig. 3 is a flowchart describing an operational process of the controller included in the patient support apparatus system;
Fig. 4 is a flowchart representation of a subroutine of the operational process of Fig. 3 describing how data is analyzed by the controller;
Fig. 5 is a flowchart representation of an alternative subroutine of the operational process of Fig. 3 describing how data is analyzed by the controller; and
Fig. 6 is a flowchart representation of another subroutine of the operational process of Fig. 3 describing how the hospital information system is notified by the controller.

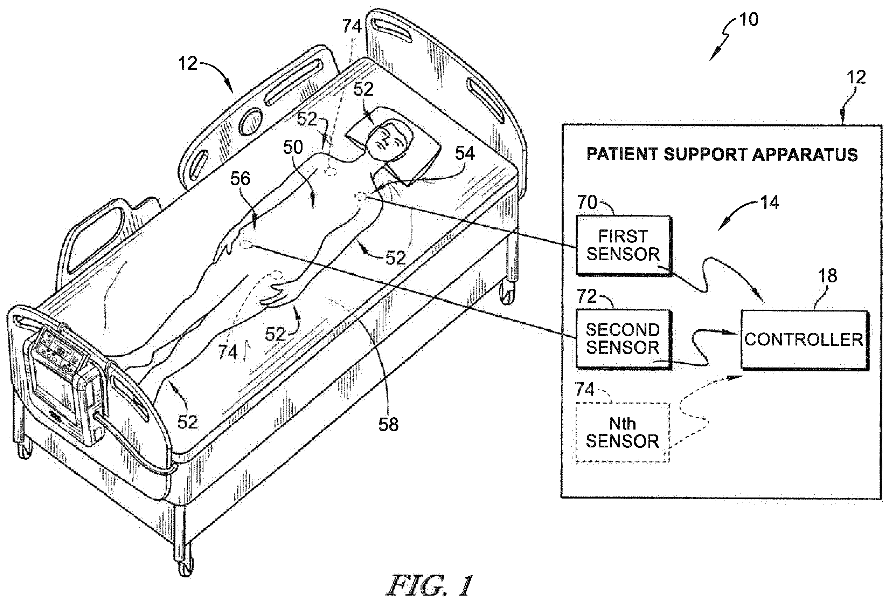

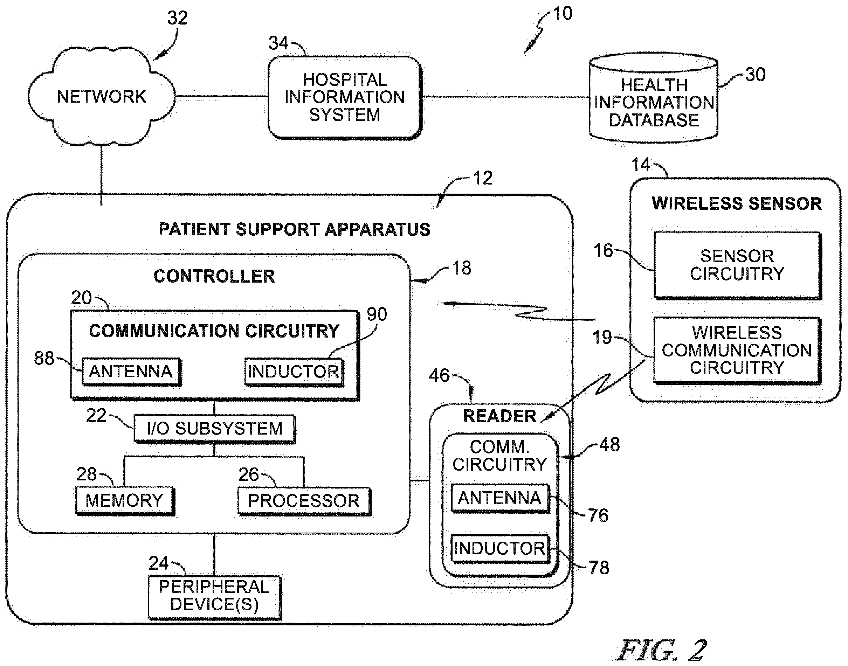

A system 10 for preventing pressure ulcers from developing on a patient 50 supported on a patient support apparatus 12 is shown in Figs. 1 and 2. The system 10 includes the patient support apparatus 12 and a plurality of body-mounted sensors 14 situated on the patient 50. The patient support apparatus 12 includes a controller 18 in communication with the body-mounted sensors 14 to monitor the patient's position and orientation relative to the patient support apparatus 12.

Typically, the patient 50 supports their body on the patient support apparatus 12 by resting on pressure areas 52 such as, for example, their head, shoulders, elbows, hips, and heels. The controller 18 is configured to generate an alarm signal if the patient 50 supports their body with the same pressure areas 52 over an extended period of time such that the patient 50 has an increased risk of developing pressure ulcers in those areas.

After resting on the same pressure area 52 for an extended period of time, tissue of the patient 50 surrounding the pressure area 52 may experience poor blood perfusion. If the patient does not periodically relieve the pressure area 52, the poor blood perfusion may increase the probability that pressure ulcers will occur in the tissue surrounding the pressure area 52. The pressure areas 52 may be relieved by periodically alternating the pressure areas 52 that support the patient 50. The pressure areas 52 may be alternated, for example, when the patient's body is repositioned under their own influence or with the aid of a caregiver or a patient rotation system 58 included in the patient support apparatus 12.

Sometimes, repositioning the patient's body does not relieve each of the pressure areas 52 on which the patient has been resting. As a result, those pressure areas 52 remain at risk for developing pressure ulcers. The system 10 is configured to monitor the patient's movement to determine whether the relative movement of the patient 50 is indicative of alternating and relieving the pressure areas 52 or if the movement is inadequate such that the patient 50 has an increased risk of developing pressure ulcers.

The body-mounted sensors 14 are configured to gather information indicative of the patient's movement and transfer the information to the controller 18 of the patient support apparatus 12 as suggested in Fig. 1. In the illustrative embodiment, the sensors 14 include accelerometers and near field communication protocols. Illustratively, each sensor 14 is coupled to the patient 50 near a pressure area 52 as suggested in Fig. 1. The pressure areas 52 may include the back of the head, ear, cheek, shoulder, ribs, breasts, elbows, genitals, sacrum, hip, knees, toes, heels, etc. of the patient 11.

The controller 18 is configured to determine a position of the sensors 14 and, thus, a position of the pressure areas 52 of the patient 50 based on the measured accelerations. The controller 18 is configured to monitor the sensors 14 to determine relative movement of the patient 50 over time. In some embodiments, the system 10 continuously monitors the sensors 14. In some embodiments, the system 10 monitors the sensors 14 in periodic intervals. In some embodiments, the periodic intervals are predetermined and regular. In some embodiments, the periodic intervals are random.

In the illustrative embodiment, the sensors 14 include a first sensor 70 coupled to a shoulder 54 of the patient 50 and a second sensor 72 coupled to a hip 56 of the patient 50 as shown in Fig. 1. The position of the patient's body may be generally defined by an orientation of a shoulder line and a hip line of the patient 50. The controller 18 is configured to determine an orientation of the shoulder line and hip line of the patient 50 based on the measurements from the sensors 70, 72. In some embodiments, the sensors 14 include a plurality of nth sensors 74 and the controller 18 is configured to determine a position of the patient based on measurements including the nth sensors 74 as suggested in Fig. 1.

After the patient 50 is repositioned on the patient support apparatus 12, the sensors 70, 72 provide position change information including an angle change of the shoulder line and the hip line. If the change in position or angle is inadequate to prevent pressure ulcers from developing on the patient 50, the controller 18 generates an alarm signal to alert a caregiver, patient rotation system 58, and/or a hospital information system 34 so that the patient 50 may be repositioned. In some embodiments, the alarm signal is transmitted via a network 32.

In some embodiments, the controller 18 is configured to determine relative movement between the sensor 70 coupled to the patient's shoulder and the sensor 72 coupled to the patient's hip. As such, the controller 18 determines an angle between the shoulder line and the hip line. In other embodiments, the controller 18 is configured to determine a change in the angle of the shoulder line and the hip line relative to the patient support apparatus 12. As such, the controller 18 determines if the patient 50 has moved from lying in a supine position to lying in a lateral recumbent position or a prone position, for example, such that the pressure areas 52 are being alternated.

In the illustrative embodiment, the sensors 14 are wireless and are configured to communicate with the controller 18 wirelessly as suggested in Fig. 1. The sensors 14 include a wireless communication circuitry 19 and a sensor circuitry 16 as shown in Fig. 2. In some embodiments, the sensors 14 may be a passive device that are unpowered and are operable to be energized inductively. In other embodiments, the sensors 14 may have a small charge source, such as a battery, for example, to power the sensors 14. Various embodiments of the sensors14 may be configured for detecting additional characteristics of the patient 50 such as, for example, one more biophysical characteristics of a patient 50 such as heart rate, temperature, respiration rate, blood pressure, pulse oximetry, electrocardiographic (EKG) information, electroencephalographic (EEG), information, muscle movement, or other similar information. In other embodiments, the sensors 14 are wired and communicate with the controller 18 via wired means.

In some embodiments, the sensors 14 include pressure sensors configured to measure pressure acting on the patient 50. In some embodiments, the first sensor 70 is coupled to the patient 50 in a location that is spaced apart from a high-pressure area of the patient's shoulder. The second sensor 72 is coupled to the patient 50 in a location that is spaced apart from a high-pressure area of the patient's hip. The controller 18 is configured to use data from the first and second sensors 70, 72 as a proxy for actual pressures acting on the patient at the high-pressure areas of the patient 50.

The patient support apparatus 12 includes the controller 18 which includes communication circuitry 20 that acts as an interface to the sensors 14 as shown in Fig. 2. The controller 18 includes an I/O subsystem 22 coupled to the communication circuitry 20 as well as a processor 26 and a memory device 28. The processor 26 is operable to use instructions stored in the memory 28 to operate the I/O subsystem 22 which controls the communication circuitry 20 as well as communication with the network 32. The controller 18 is in communication with peripheral devices of the patient support apparatus such as the peripheral device 24 shown in Fig. 2.

The peripheral device 24 may be any of a number of subsystems of a patient support apparatus known in the art. For example if the patient support apparatus 12 is embodied as a hospital bed, the peripheral device 24 may include any one of a scale system, side rail position monitoring system, a brake mechanism monitoring system, a bed position monitoring system, a patient position monitoring system including bed exit detection capability, or a therapy device such as a therapeutic mattress, for example. In general, the peripheral device 24 may be embodied as any subsystem or device that monitors a patient condition, monitors and operating condition of the patient support apparatus 12, controls and operating condition of the patient support apparatus 12, or provides therapy to the patient 50 supported on the patient support apparatus 12.

It is contemplated that the controller 18 may be programmed to operate as a universal interface capable of communicating with any of a number of different sensors 14. During normal operation, the controller 18 will regularly attempt to initiate communication with a sensor 14 such that any wireless sensor within the operating range of the communication circuitry 20 of the controller 18 may be detected and engaged by the controller 18.

The patient support apparatus 12 may also include one or more separate readers 46 that are coupled to the controller 18. The readers 46 include communication circuitry 48 that communicates with the I/O subsystem 22 to share information with the controller 18. Illustratively, the reader 46 includes an antenna 76 and an inductor 78.

In the illustrative embodiment, the communication circuitry 20 includes an antenna 88 that receives the wireless signal from the sensors 14 and an inductor 90 that is operable to generate a magnetic field that generates a current in an inductor of the communication circuitry 19 of the wireless sensor 14. The communication circuitry 20 also includes a power circuit that is operable to convert the current generated in the inductor to power the communication circuitry 20 and the sensor circuitry 16 of the wireless sensor 14. The communication circuitry 20 also includes the antenna 88 that transmits signals from the communication circuitry 20 to the communication circuitry 48.

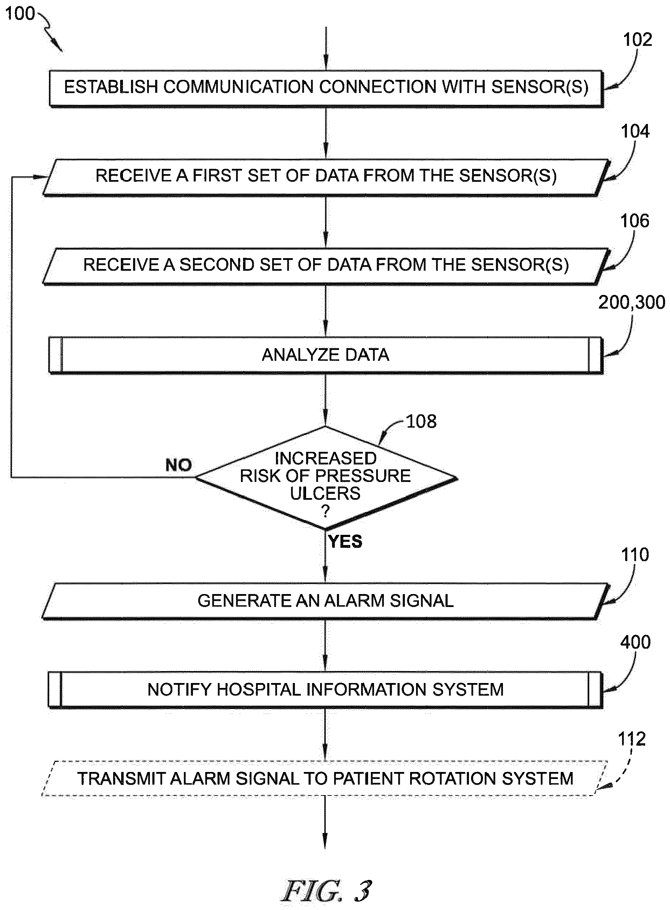

A process 100 shown in Fig. 3 provides an overview of the various actions the controller 18 may take in relation to the sensors 14. Process steps shown in phantom indicate that the particular process step is optional as will be discussed in further detail below. At the initial process step 102 the controller 18 establishes a communication connection between the wireless sensors 14 and the controller 18.

Process 100 proceeds to process step 104 where the controller 18 receives a first set of data from the sensors 70, 72 as suggested in Fig. 3. In some embodiments, the controller 18 requests the data from the sensors 70, 72. In the illustrative embodiment, the controller 18 receives data indicative of a first position of the first sensor 70 coupled to the patient's shoulder 54 and data indicative of a first position of the second sensor 72 coupled to the patient's hip 56. In other embodiments, the first set of data is indicative of pressures acting on the patient 50.

After a time interval, process 100 proceeds to process step 106 where the controller 18 receives a second set of data from the sensor 70, 72 as shown in Fig. 3. In the illustrative embodiment, the controller 18 receives data indicative of a second position of the first sensor 70 coupled to the patient's shoulder 54 and data indicative of a second position of the second sensor 72 coupled to the patient's hip 56. In some embodiments, the controller 18 receives the second set of data after a predetermined period of time. In some embodiments, the controller 18 receives the second set of data after about two hours. In some embodiments, the controller 18 is configured to receive data sets periodically and compare each of the data sets to one another.

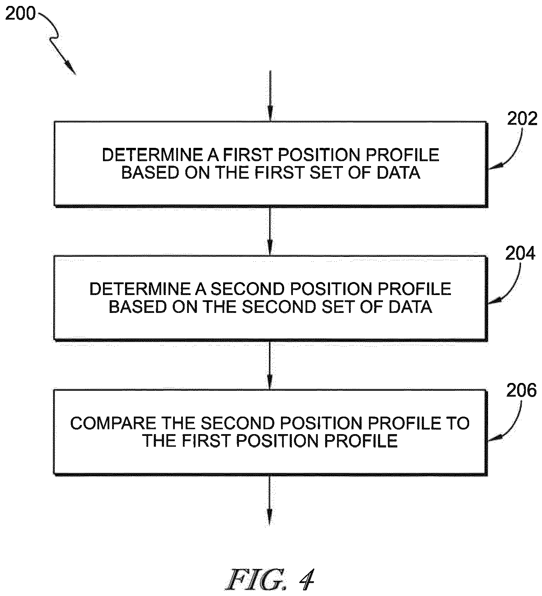

In the illustrative embodiment, process 100 proceeds to subroutine 200 in which the data sets are analyzed by the controller 18 as suggested in Figs. 3 and 4. The subroutine 200 includes a first process 202 in which the controller 18 determines a first position profile of the patient 50 based on the first set of data. In the illustrative embodiment, the first position profile includes a first shoulder line and a first hip line of the patient 50. The controller 18 is configured to determine an orientation of the shoulder line and the hip line of the patient 50 based on the measurements from the sensors 70, 72.

Subroutine 200 proceeds to process 204 in which the controller 18 determines a second position profile of the patient 50 based on the second set of data as suggested in Fig. 4. In the illustrative embodiment, the second position profile includes a second shoulder line and a second hip line of the patient 50.

Subroutine 200 proceeds to process 206 in which the controller 18 compares the second position profile to the first position profile as suggested in Fig. 4. As such, the controller 18 can determine if the patient 50 has been repositioned on the patient support apparatus 12 during the time interval between the first and second data sets. In some embodiments, the controller 18 is configured to determine a position profile after each data set is received.

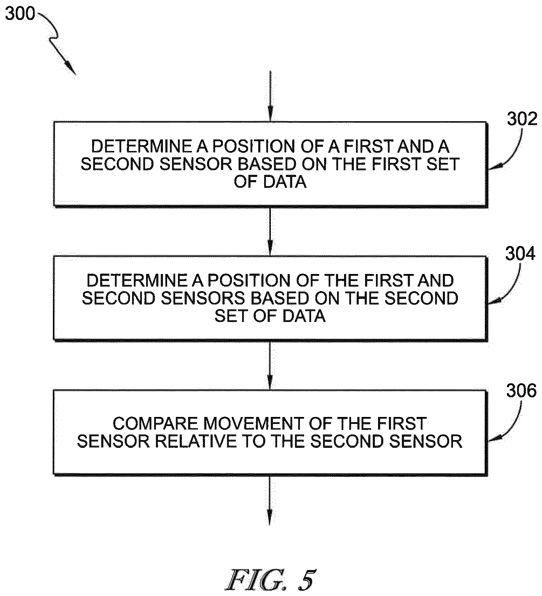

In some embodiments, the process 100 proceeds to subroutine 300 instead of subroutine 200 as suggested in Figs. 3 and 5. In an initial step 302 of subroutine 300, the controller 18 determines a first position of the first sensor 70 and a first position of the second sensor 72 based on the first set of data. Subroutine 300 proceeds to process 304 in which the controller 18 determines a second position of the first sensor 70 and a second position of the second sensor 72 based on the second set of data. Subroutine 300 proceeds to process 306 in which the controller 18 compares the second position of the first and second sensors 70, 72 to the first position of the first and second sensors 70, 72. As such, the controller 18 can determine if the patient 50 has been repositioned on the patient support apparatus 12 during the time interval between the first and second data sets.

Process 100 proceeds to decision step 108 where the controller 18 evaluates whether the analysis of subroutine 200, 300 indicates that the patient 50 has an increased risk for developing pressure ulcers. If the controller 18 determines that the patient 50 is at an increased risk of developing pressure ulcers, process 100 proceeds to process step 110. If the controller 18 determines that the patient 50 is not at an increased risk of developing pressure ulcers, then process 100 loops back and continues to monitor for relevant data.

In particular, the controller 18 determines if the change in the first and second position profile is inadequate to prevent pressure ulcers from developing. As an example, if the change in the orientation of the shoulder and hip lines is relatively small, the controller 18 determines that the patient 50 is supporting themselves on the same pressure areas over the elapsed time period between data sets. As such, the controller 18 determines that the patient 50 should be repositioned to relieve the pressure areas. In the illustrative embodiment, the controller 18 determines that the patient 50 is at an increased risk of developing pressure ulcers if the patient 50 has not been adequately repositioned within a two hour time interval.

In process step 110, the controller 18 generates an alarm signal in response to an affirmative decision in decision step 108 as suggested in Fig. 3. In some embodiments, the alarm signal causes the patient support apparatus 12 to notify a caregiver. In some embodiments, the alarm signal is transmitted to the hospital information system 34 via the hospital network 32. In some embodiments, the alarm signal is used by the patient support apparatus 12 to cause the patient rotation system 58 to reposition the patient 50.

In the illustrative embodiment, process 100 then proceeds to subroutine 400 were the controller 18 is operable to notify the hospital information system 34 of information related to the sensors 14. The subroutine 400 is operable to associate information regarding the sensors14 with the patient to which the sensors 14 are applied and the patient support apparatus 12 so that the information may be properly placed in the health information database 30 and associated with the particular patients electronic medical record.

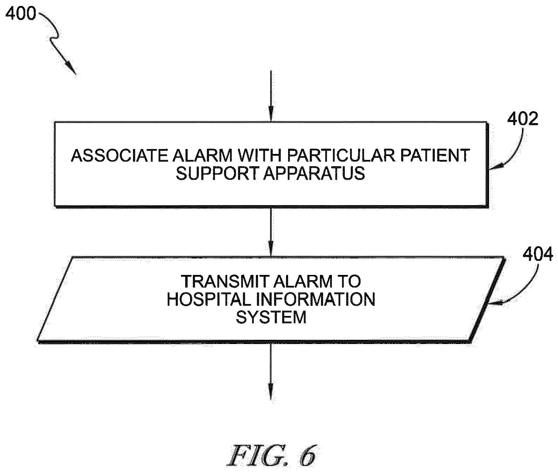

Subroutine 400 includes a process step 402 in which the controller 18 is operable to identify the patient support apparatus 12 and include information in the alarm signal that associates the patient 50 with the patient support apparatus 12 as suggested in Fig. 6. The controller 18 may optionally receive a unique identifier from the patient support apparatus 12. Once the relationship between the patient 50 and the patient support apparatus 12 is established, the controller 18 transmits the alarm to the hospital information system 34 via the hospital network 32 in process step 404.

Once subroutine 400 is completed process 100 continues to the optional process step 112. In process step 112, the alarm signal is transmitted to the patient rotation system 58 to cause the patient rotation system 58 to reposition the patient 50. Process 100 then loops back and continues to monitor for relevant data.

Information may be transferred over the network 32 to the hospital information system 34 by the controller 18 in real time, or may be stored in memory 28 and transferred to the network 32 on an intermittent basis. In still other embodiments, when the information is stored on the controller 18, the hospital information system 34 may be operable to query the controller 18 to receive the most recent information stored by controller 18 in memory 28. Controller 18 may combine and associate information from peripheral devices 24 as well as wireless sensor 14 so that all of the information may be transferred to the hospital information system 34 as a single record. It should be understood that the network 32 may be connected to the patient support apparatus 12 through a wired data link, or the network connection may be a wireless data link.

Although certain illustrative embodiments have been described in detail above, variations and modifications exist.

## Claims

1. A system for preventing pressure ulcers on a patient supported on a patient support apparatus, the system comprising:
a first sensor configured to be coupled to the patient at a first location,
a second sensor configured to be coupled to the patient at a second location, and
means for comparing movement of the first sensor relative to the second sensor over time to determine if the relative movement of the first and second sensors is indicative of a change in an orientation of the patient or an orientation of the patient support apparatus.

2. The system of claim 1, wherein the means for comparing movement of the first sensor relative to the second sensor includes a controller including a processor and a memory, the memory having stored therein a plurality of instructions that when executed by the processor cause the controller to:
determine a first position of the first sensor and a first position of the second sensor,
determine a second position of the first sensor and a second position of the second sensor,
compare the change in position of the first sensor relative to the change in position of the second sensor to determine if the patient has moved relative to the patient support apparatus during a predetermined time interval, and
generate an alarm signal if the relative movement of the patient is indicative of an increased risk of the patient developing pressure ulcers due to inadequate movement of the patient during the predetermined time interval.

3. The system of claim 2, wherein the first sensor includes an accelerometer coupled to a hip of the patient.

4. The system of claim 3, wherein the second sensor includes an accelerometer coupled to a shoulder of the patient.

5. The system of any one of claims 2 to 4, wherein the first sensor is configured to be coupled to the patient in a location that is spaced apart from a high-pressure area of the patient's hip, the second sensor is configured to be coupled to the patient in a location that is spaced apart from a high-pressure area of the patient's shoulder, and the plurality of instructions further cause the controller to use data from the first and second sensors as a proxy for actual pressures acting on the patient at the high-pressure areas of the patient.

6. The system of any one of claims 2 to 5, wherein the plurality of instructions further cause the controller to transmit the alarm signal to the patient support apparatus to activate a turn system included in the patient support apparatus to cause the turn system to reposition the patient relative to the patient support apparatus.

7. The system of any one of claims 2 to 6, wherein the controller is connected to a hospital network and the plurality of instructions further cause the controller to transmit the alarm signal over the hospital network to alert a caregiver.

8. A controller of a patient support apparatus comprising:
a processor, and
a memory, the memory having stored therein a plurality of instructions that when executed by the processor cause the controller to:
receive a first data set indicative of a first position of a patient supported on the patient support apparatus relative to the patient support apparatus,
receive a second data set indicative of a second position of the patient relative to the patient support apparatus after a time interval,
compare the second position of the patient to the first position of the patient, and
generate an alarm signal if a difference between the second position of the patient and the first position of the patient is indicative of an increased risk of the patient developing pressure ulcers due to inadequate movement of the patient's limbs over the time interval.

9. The controller of claim 8, wherein each of the first and second data sets include information indicative of a position of the patient's hip and shoulder relative to the patient support apparatus.

10. The controller of either claim 8 or claim 9, wherein the controller is configured to receive data from at least one sensor that is configured to be coupled to the patient in a location that is spaced apart from a high-pressure area of the patient and the plurality of instructions further cause the controller to use the data from the at least one sensor as a proxy for a pressure acting on the patient at the high-pressure area.

11. The controller of claim 10, wherein the high-pressure area of the patient includes one or more of the patient's hip and the patient's shoulder.

12. The controller of either claim 10 or claim 11, wherein the plurality of instructions further cause the controller to transmit the alarm signal to the patient support apparatus to activate a turn system included in the patient support apparatus to cause the turn system to reposition the patient relative to the patient support apparatus.

13. The controller of any one of claims 8 to 12, wherein the time interval is about two hours.

14. The controller of any one of claims 8 to 13, wherein the controller is connected to a hospital network and the plurality of instructions further cause the controller to transmit the alarm signal over the hospital network to alert a caregiver.

15. The controller of any one of claims 8 to 14, wherein comparing the second position of the patient to the first position of the patient includes comparing movement of a first sensor relative to a second sensor over time to determine if the relative movement of the first and second sensors is indicative of a change in an orientation of the patient.
